Europäisches Patentamt

European Patent Office

Office européen des brevets

(11) Publication number: **0 147 932**
**B1**

# EUROPEAN PATENT SPECIFICATION

(45) Date of publication of patent specification: **14.06.89**

(51) Int. Cl.⁴: **A 61 K 9/22,** A 23 K 1/00

(21) Application number: **84307800.7**

(22) Date of filing: **12.11.84**

(54) **Water soluble glass compositions.**

(30) Priority: **26.11.83 GB 8331661**

(43) Date of publication of application:
**10.07.85 Bulletin 85/28**

(45) Publication of the grant of the patent:
**14.06.89 Bulletin 89/24**

(84) Designated Contracting States:
**AT BE CH DE FR IT LI LU NL SE**

(56) References cited:
**EP-A-0 009 338**
**EP-A-0 013 077**
**EP-A-0 080 835**
**GB-A-2 079 152**
**GB-A-2 081 703**

(73) Proprietor: **STC PLC**
**10, Maltravers Street**
**London, WC2R 3HA (GB)**

(72) Inventor: **Drake, Cyril Francis**
**1 Church cottages Peldon Road**
**Harlow Essex (GB)**
Inventor: **Brocklehurst, John Robert**
**26A Thorley Park Road**
**Bishop's Stortford Herts, CM23 2NQ (GB)**

(74) Representative: **Ryan, John Peter William**
**STC Patents West Road**
**Harlow Essex CM20 2SH (GB)**

Courier Press, Leamington Spa, England.

## Description

This invention relates to therapeutic devices incorporating water soluble glass compositions whose dissolution rate is strongly dependent on the pH of a surrounding aqueous medium. Water-soluble glass compositions are finding a variety of uses in medical and veterinary applications for the controlled release of one or more active materials. There are special circumstances and particular types of product where it is desirable that the rate of solution of a glass should change rapidly when the pH of the aqueous medium is changed. For example, where a pharmaceutical product is to be delivered orally, e.g. as a tablet or bolus, or as a granular material, it is desirable that none should be delivered during the transport of the material from mouth to stomach but that the product should be delivered rapidly as soon as the product reaches the stomach.

We have found that it is possible to select compositions of water-soluble phosphate glass whose dissolution rates show a strong dependence on the pH conditions.

According to one aspect of the invention there is provided a therapeutic device for oral administration to the alimentary tract, the device being readily soluble in that part of the alimentary tract where low pH (acid) conditions pertain, but which is of low solubility in that part of the tract where high pH conditions pertain, the device comprising a water soluble glass composition, the dissolution rate of which is a function of the pH condition of an aqueous medium in contact with the device, wherein the glass includes phosphorus pentoxide as a glass forming oxide, and a mixture of metal oxides including sodium oxide, said mixture of oxides constituting the glass modifying oxide content of the glass, characterised in that the glass composition comprises one of the following:—

(i) 40.0 mole percent sodium oxide, 35.6 mole percent phosphorus pentoxide and 24.1 mole percent cupric oxide;

(ii) 25 mole percent sodium oxide, 44.6 mole percent phosphorous pentoxide and 30.2 mole percent calcium oxide;

(iii) 36.4 mole percent sodium oxide, 33.6 mole percent phosphorous pentoxide, 10.0 mole percent zinc oxide and 20.0 mole percent cupric oxide;

(iv) 30.0 mole percent sodium oxide, 45.0 mole percent phosphorous pentoxide, and 25.0 mole percent calcium oxide;

(v) 33.0 mole percent sodium oxide, 41.2 mole percent phosphorous pentoxide, 25.7 mole percent magnesium oxide and 1.5 mole percent aluminium oxide.

(vi) 33.0 mole percent sodium oxide, 33.0 mole percent phosphorous pentoxide and 45.0 mole percent magnesium oxide.

The composition of the glasses is herein described in terms of the mole percent of the normal oxides of the elements which they contain. This method of describing the composition does not imply that these oxides are used to prepare the glass batch, as other compounds of the elements such as carbonate, sulphate, hydroxide, etc. which decompose to the oxide at the temperature of the glass melt may optionally be used in the batch. Nor does the method of describing the composition necessarily imply that such oxides are present as discrete molecules of the oxides in the glass.

The constituent oxides in the glass will be classified, conventionally, as glass-forming oxides and glass-modifying oxides. In the case of the glasses disclosed here, the glass-forming oxide is $P_2O_5$ and the glass-modifying oxides are alkali-metal oxides ($M_2O$), alkaline earth metal oxides (MO), other divalent metal oxides (MO) and the metal sesquioxides ($M_2O_3$), such as $Al_2O_3$ or $Fe_2O_3$. A small proportion of $P_2O_5$ may be replaced by another glass-forming oxide such as $SiO_2$, $SO_3$, $B_2O_3$, or $SeO_2$ without substantially changing the behaviour of the glass. A small proportion of the glass-modifying oxide may be a metal oxide not covered by those defined above, e.g. to provide trace elements. A small proportion of fluoride, chloride, bromide or iodide ions and/ or a small amount of a precious metal such as Au or Pt, which is probably present as colloidal or atomic zero-valent particles, may be included.

Embodiments of the invention will now be described with reference to the accompanying figures in which:

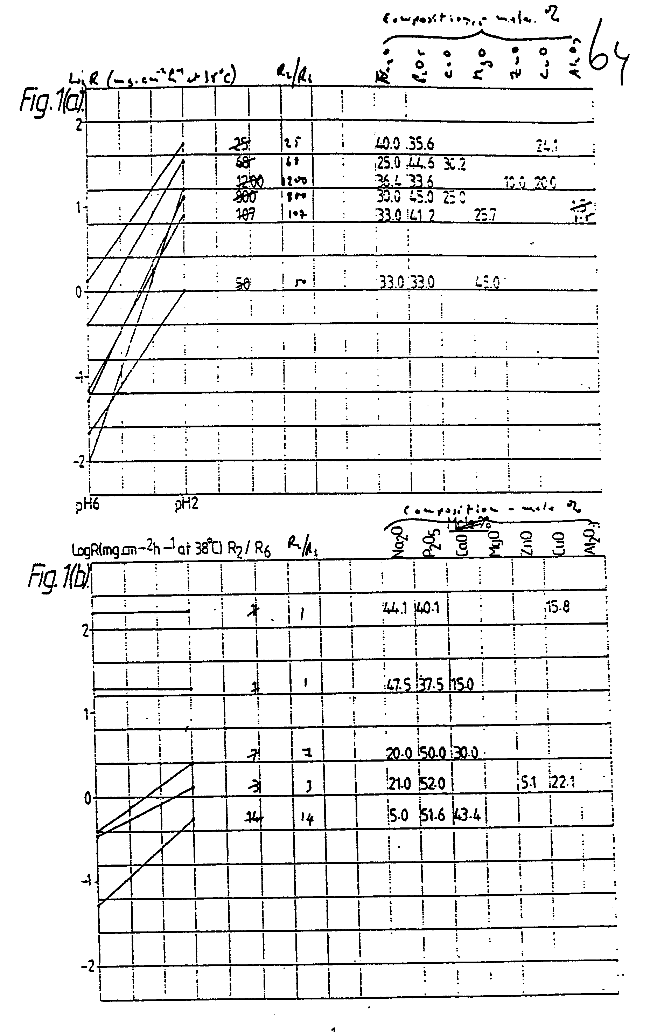

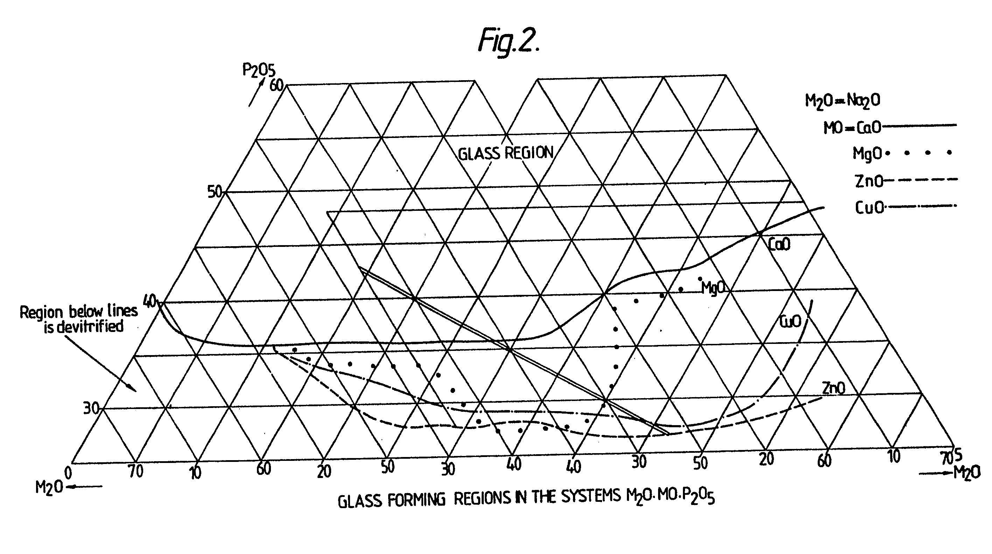

Figs. 1(a+b) show typical relationships between glass dissolution rate and solution pH both for the glass compositions of the present invention (Fig. 1a) and for conventional compositions for comparative purposes (Fig. 1b).

The glasses are formed by melting their constituent materials in oxide form or in the form of compounds which decompose on heating to form oxides, to obtain an homogeneous melt. This melt is then cast and may be granulated. As some phosphorus pentoxide is lost during melting, we prefer to determine the glass composition by chemical analysis of the finished material.

The pH-dependence of the solution rate of the glasses has been measured by placing small specimens of the glass in an open gauge basket in flowing solutions of various pH values and determining the weight loss with time. Some typical results are shown in Fig. 1, the compositions being expressed in mole percentage of oxides. In Fig. 1, part a relates to the glasses of the present invention whilst the compositions of part b are shown for comparative purposes. In Fig. 1, the factor R is the glass dissolution rate measured in $mg \cdot cm^{-2} \cdot h^{-1}$ at a temperature of 38°C. As can be seen from Fig. 1, the glasses of part a show a significant dissolution rate dependence on solution pH.

We have found that generally in glasses

$$xM_2O \cdot y(M,M)O \cdot (100-x-y)P_2O_5,$$

where x and y are the molar percentages of $M_2O$ and (M,M)O respectively, a decrease in the mole

percentage of $P_2O_5$ and a decrease in the mole percentage of $M_2O$ in the glass increases the pH-dependence of the solution rate.

Moreover, in glasses with a given value of x and y, additions of small amounts of $M_2O_3$ increase substantially the pH-dependence of the solution rate.

It will be clear that by selection of the composition in terms of the three variables, (mole %) $P_2O_5$, $M_2O$, and $M_2O_3$, it is possible to find a composition which combines a chosen absolute level of solution rate with a high pH dependence of solution rate.

The limits of the composition ranges which can be used are, of course, ultimately set by the limits of composition which will form glasses on cooling from the melt. It is well known to those skilled in the art that a glass-forming region is usually extended by the addition of more constituents to a given system. If additional constituents are added, some adjustments, which can be determined experimentally will have to be made to the glass-forming limits, but we have found that the limits of the composition ranges, which have a large change of solution rate with pH, are not sensibly altered.

The glasses may be used together with a polymeric resin in the preparation of composite materials e.g. for use in animal feed additives. The composite may be formed by compaction of the powdered glass, a polymeric resin and particulate inert material on which an active material is dispersed. The composite may be formed into a tablet or bolus. Such composite materials are described in our published specifications EP—A—0 080 835 and EP—A—0 080 330. If an active material is enclosed in a coating all or part of which is a glass the rate of solution of which is two orders of magnitude greater at pH 1—2 than at pH 6—8 then, as the human stomach has a pH 1—2, if the composition of the glass is selected so that it will dissolve completely in one hour at pH 6, it will dissolve completely in less than one minute in the stomach so releasing the contained pharmaceutical. Other applications of the glasses include rumen boluses. In particular a granular material or tablet incorporating the glass may include a pharmaceutical or an animal nutrition supplement which is required to pass through the rumen and be delivered in the lower-pH, post-rumen region of the alimentary tract of a ruminant.

## Claims

1. A therapeutic device for oral administration to the alimentary tract, the device being readily soluble in that part of the alimentary tract where low pH (acid) conditions pertain, but which is of low solubility in that part of the tract where high pH conditions pertain, the device comprising a water soluble glass composition, the dissolution rate of which is a function of the pH condition of an aqueous medium in contact with the device, wherein the glass includes phosphorus pentoxide as a glass forming oxide, and mixture of metal oxides including sodium oxide, said mixture of oxides constituting the glass modifying oxide content of the glass, characterised in that the glass composition comprises one of the following:—

(i) 40.0 mole percent sodium oxide, 35.6 mole percent phosphorous pentoxide and 24.1 mole percent cupric oxide;

(ii) 25 mole percent sodium oxide, 44.6 mole percent phosphorous pentoxide and 30.2 mole percent calcium oxide;

(iii) 36.4 mole percent sodium oxide, 33.6 mole percent phosphorous pentoxide, 10.0 mole percent zinc oxide and 20.0 mole percent cupric oxide;

(iv) 30.0 mole percent sodium oxide, 45.0 mole percent phosphorous pentoxide, and 25.0 mole percent calcium oxide;

(v) 33.0 mole percent sodium oxide, 41.2 mole percent phosphorous pentoxide, 25.7 mole percent magnesium oxide and 1.5 mole percent aluminium oxide.

(vi) 33.0 mole percent sodium oxide, 33.0 mole percent phosphorous pentoxide and 45.0 mole percent magnesium oxide.

2. A device as claimed in claim 1, characterised in that it incorporates an animal feed additive.

3. A device as claimed in claim 2, characterised in that the feed additive is methionine.

## Patentansprüche

1. Therapeutisches Mittel zur oralen Zuführung an den Ernährungstrakt, wobei das Mittel sehr leicht in dem Teil des Ernährungstrakts lösbar ist, in dem niedrige pH-(saure) Bedingungen vorherrschen, jedoch eine geringe Löslichkeit in dem Teil des Ernährungstraktes aufweist, in dem Bedingungen mit hohem pH-Wert vorherrschen, wobei das Mittel eine wasserlösliche Glaszusammensetzung umfaßt, dessen Lösungsgeschwindigkeit eine Funktion des pH-Zustandes eines wässrigen Mediums ist, das mit dem Mittel in Berührung steht, worin das Glas Phosphorpentoxid als glasbildendes Oxid und eine Mischung von Metalloxiden unter Einschluß von Natriumoxid einschließt, und wobei die Mischung von Oxiden den glasmodifizierenden Oxidanteil des Glases bildet, dadurch gekennzeichnet, daß die Glaszusammensetzung eines der folgenden Bestandteile umfaßt:

(i) 40,0 Molprozent Natriumoxid, 35,6 Molprozent Phosphorpentoxid und 24,1 Molprozent Cuprioxid;

(ii) 25 Molprozent Natriumoxid, 44,6 Molprozent Phosphorpentoxid und 30,2 Molprozent Kalziumoxid;

(iii) 36,4 Molprozent Natriumoxid, 33,6 Molprozent Phosphorpentoxid, 10,0 Molprozent Zinkoxid und 20,0 Molprozent Cuprioxid;

(iv) 30,0 Molprozent Natriumoxid, 45,0 Molprozent Phosphorpentoxid und 25,0 Molprozent Kalziumoxid;

(v) 33,0 Molprozent Natriumoxid, 41,2 Molprozent Phosphorpentoxid, 25,7 Molprozent Magnesiumoxid und 1,5 Molprozent Aluminiumoxid;

(vi) 33,0 Molprozent Natriumoxid, 33,0 Molprozent Phosphorpentoxid und 45,0 Molprozent Magnesiumoxid.

2. Mittel nach Anspruch 1, dadurch gekennzeichnet, daß es einen Tiernahrungsmittelzusatz enthält.

3. Mittel nach Anspruch 2, dadurch gekennzeichnet, daß der Nahrungsmittelzusatz Methionin ist.

**Revendications**

1. Dispositif thérapeutique pour administration orale au tube digestif, le dispositif étant facilement soluble dans la partie du tube digestif dans laquelle règne de basses conditions de pH (acides), mais ayant une faible solubilité dans la partie du tube digestif dans laquelle règnent des conditions de pH élevé, le dispositif comprenant une composition de verre, soluble dans l'eau et dont la vitesse de dissolution est fonction de l'état de pH d'un milieu aqueux en contact avec le dispositif, le verre comprenant du pentoxyde de phosphore comme oxyde formateur de verre, et un mélange d'oxydes de métaux comprenant l'oxyde de sodium, ledit mélange d'oxydes constituant les oxydes modificateurs du verre contenus dans le verre, dispositif caractérisé en ce que la composition du verre comprend l'une des compositions suivantes:

(i) 40,0 moles % d'oxyde de sodium, 35,6 moles % de pentoxyde de phosphore et 24,1 moles % d'oxyde cuivrique;

(ii) 25 moles % d'oxyde de sodium, 44,6 moles % de pentoxyde de phosphore et 30,2 moles % d'oxyde de calcium;

(iii) 36,4 moles % d'oxyde de sodium, 33,6 moles % de pentoxyde de phosphore, 10,0 moles % d'oxyde de zinc et 20,0 moles % d'oxyde cuivrique;

(iv) 30,0 moles % d'oxyde de sodium, 45,0 moles % de pentoxyde de phosphore et 25,0 moles % d'oxyde de calcium;

(v) 33,0 moles % d'oxyde de sodium, 41,2 moles % de pentoxyde de phosphore, 25,7 moles % d'oxyde de magnésium et 1,5 mole % d'oxyde d'aluminium;

(vi) 33,0 moles % d'oxyde de sodium, 33,0 moles % de pentoxyde de phosphore et 45,0 moles % d'oxyde de magnésium.

2. Dispositif tel que revendiqué à la revendication 1, caractérisé en ce qu'il incorpore un additif pour l'alimentation des animaux.

3. Dispositif tel que revendiqué à la revendication 2, caractérisé en ce que l'additif pour alimentation est de la méthionine.

Fig. 1(a)

Log R (mg.cm⁻².h⁻¹ at 38°C)   R₂/R₁   Composition - mole %

$Na_2O$  $P_2O_5$  $CaO$  $MgO$  $ZnO$  $CuO$  $Al_2O_3$

| | R₂/R₁ | Na₂O | P₂O₅ | CaO | MgO | ZnO | CuO | Al₂O₃ |
|---|---|---|---|---|---|---|---|---|
| 25 | 25 | 40.0 | 35.6 | | | | | 24.1 |
| 68 | 68 | 25.0 | 44.6 | 30.2 | | | | |
| 1200 | 1200 | 36.4 | 33.6 | | | 10.0 | 20.0 | |
| 800 | 800 | 30.0 | 45.0 | 25.0 | | | | |
| 107 | 107 | 33.0 | 41.2 | | 25.7 | | | |
| 50 | ~ | 33.0 | 33.0 | | 45.0 | | | |

pH6   pH2

Fig. 1(b)

Log R(mg cm⁻²h⁻¹ at 38°C)  R₂ / R₆   R₂/R₁   Composition - mole %
Mole %

$Na_2O$  $P_2O_5$  $CaO$  $MgO$  $ZnO$  $CuO$  $Al_2O_3$

| | R₂/R₆ | R₂/R₁ | Na₂O | P₂O₅ | CaO | MgO | ZnO | CuO | Al₂O₃ |
|---|---|---|---|---|---|---|---|---|---|
| | 1 | 1 | 44.1 | 40.1 | | | | 15.8 | |
| | 1 | 1 | 47.5 | 37.5 | 15.0 | | | | |
| | 7 | 7 | 20.0 | 50.0 | 30.0 | | | | |
| | 3 | 3 | 21.0 | 52.0 | | | 5.1 | 22.1 | |
| | 14 | 14 | 5.0 | 51.6 | 43.4 | | | | |

1

Fig.2.

GLASS FORMING REGIONS IN THE SYSTEMS $M_2O \cdot MO \cdot P_2O_5$